# FASCICULE DE BREVET EUROPEEN

(11) **EP 0 870 504 B1**
(45) Date de publication et mention de la délivrance du brevet: **09.03.2005**
(21) Numéro de dépôt: 98480017.7
(22) Date de dépôt: 23.03.1998
(51) Int. Cl.: C07F 9/40, A61K 31/695, A61P 19/02

(54) **Compositions à base de silicium biologiquement actif**
Zusammensetzungen enthaltendes biologisch aktives Silizium
Compositions comprising biologically active silicon

(30) Priorité: 24.03.1997 FR 9703793
(43) Date de publication de la demande: 14.10.1998
(73) Titulaire: Seguin, Marie-Christine, 98000 Monaco (MC); Gueyne, Jean, 98000 Monaco (MC)
(72) Inventeur: Seguin, Marie-Christine, 98000 Monaco (MC); Gueyne, Jean, 98000 Monaco (MC)
(74) Mandataire: Bonneau, Gérard

(56) Documents cités:
- EP-A- 0 525 573
- WO-A-96/10574
- WO-A-96/10575
- FR-A- 1 177 091
- FR-A- 2 725 207
- FR-M- 6 871
- HENROTTE J G ET AL: "Le rôle regulateur du silicium dans la division cellulaire" C. R. ACAD. SCI., SER. 3 (CRASEV,07644469);88; VOL.306 (17); PP.525-8, XP002048854 CNRS;FAC. PHARM.; PARIS; 75006; FR. (FR)

## Description

La présente invention a pour objet de nouvelles compositions pharmaceutiques agissant par inhibition des sérines protéases et comprenant au moins un composé à base de silicium biologiquement actif.

Les composés à base de silicium biologiquement actif sont des composés organo-siliciés et plus particulièrement des silanols présentant plusieurs groupements Si-OH. Les silanols sont décrits dans l'état de la technique comme constituant une forme de silicium assimilable par l'organisme, à condition de posséder la propriété d'exister en solution aqueuse sous forme d'oligomères solubles de faible poids moléculaire(EP-0 289 366).

Plusieurs complexes de silanols ayant des propriétés thérapeutiques intéressantes ont déjà été décrits dans le passé. Le brevet spécial de médicament N° 6.871 M déposé le 23 décembre 1966 décrit un complexe organo-silicique utile comme médicament contre tous les phénomènes inflammatoires. FR-A-2725207 décrit des précurseurs de silanols qui sont des composés à base de silicium possédant des liaisons biologiquement hydrolysables notamment au contact des tissus vivants et permettant la libération des oligomères possédant les fonctions Si-OH biologiquement actives.
Les silanols ainsi obtenus *in vivo* sont utiles en thérapeutique, notamment pour le traitement de l'arthrose, pour la restructuration des microvaisseaux, pour le traitement des tendinites des sportifs, pour le traitement de l'asthénie, pour le traitement de la cataracte, pour le traitement des artériopathies tel que notamment l'artériosclérose et plus généralement pour leur activité anti-inflammatoire, anti-oedemateuse, anti-glycation, anti-radicalaire, analgésique, régénératrice et réparatrice.

Les silanols de l'art antérieur sont administrés par voie générale, orale ou parentérale, et agissent d'une manière non spécifique.

Pour notamment remédier à cet inconvénient, le but principal de la présente invention est de proposer une composition pharmaceutique agissant par inhibition des sérines protéases, comprenant un composé à base de silicium biologiquement actif capable d'atteindre dans l'organisme un tissu ou un organe spécifique, notamment les tissus cartilagineux, et d'exprimer au sein de ces tissus une activité pharmacologique.

La composition pharmaceutique selon l'invention comprend, en association avec tout excipient approprié, au moins un composé de formule générale (I) suivante :

R₆R₇P (O) - (CHR₅)ₘ - (CHR₄)ₙ - Si (OR₁) (OR₂) (R₃) (I)

dans laquelle :
R₁ et R₂ chacun indépendamment représentent un atome d'hydrogène ou un groupement alkyle,
R₃ représente un groupement hydroxyle ou un groupement alcoxy ou un groupement alkyle,
R₄ et R₅ représentent chacun indépendamment un atome d'hydrogène, un groupement hydroxyle, un groupement alkyle ou une fonction acide carboxylique,
R₆ et R₇ représentent chacun indépendamment un groupement alcoxy,
n est un entier supérieur ou égal à 0 et inférieur ou égal à 4,
m est un entier strictement supérieur à 0 et inférieur ou égal à 3.

Avantageusement, la composition pharmaceutique selon l'invention comprend un composé répondant à la formule (I) dans laquelle :
R₁ et R₂ chacun indépendamment représentent un atome d'hydrogène ou un groupement alkyle,
R₃ représente un groupement hydroxyle ou un groupement alcoxy ou un groupement alkyle,
R₄ et R₅ représentent chacun indépendamment un atome d'hydrogène ou un groupement hydroxyle,
R₆ et R₇ représentent chacun indépendamment un groupement alcoxy,
n est un entier supérieur ou égal à 0 et inférieur ou égal à 4,
m est un entier strictement supérieur à 0 et inférieur ou égal à 3,
et la somme (m + n) est strictement inférieure à 6.

Parmi ces composés, sont préférés les composés de formule (I) dans laquelle :
R₁ et R₂ chacun indépendamment représentent un atome d'hydrogène ou un groupement C₁₋₄ alkyle,
R₃ représente un groupement hydroxyle ou un groupement alcoxy ou un groupement C₁₋₄ alkyle,
R₄ et R₅ sont chacun indépendamment un atome d'hydrogène ou un groupement hydroxyle,
R₆ et R₇ représentent chacun indépendamment un groupement C₁₋₄ alcoxy,
n est un entier supérieur ou égal à 0 et inférieur ou égal à 4,
m est un entier strictement supérieur à 0 et inférieur ou égal à 3,
et la somme (m + n) est égale à 2.

Suivant un mode de réalisation préféré de l'invention, le composé de formule (I) entrant dans la composition pharmaceutique selon l'invention est le diéthylphosphatoethyltriéthoxysilane.

Avantageusement, la composition pharmaceutique selon l'invention est administrée par voie orale, rectale ou topique.

Suivant un mode de réalisation particulier, la composition pharmaceutique selon l'invention est sous forme solide.

Suivant un autre mode de réalisation particulier, l'excipient comprend plusieurs fonctions hydroxyles. De préférence, l'excipient est le lactose.

Le but de l'invention est de proposer un médicament destiné au traitement de l'arthrose, de la polyarthrite chronique évolutive, agissant par inhibition des sérines protéases, qui comprenne la composition pharmaceutique ci-dessus décrite.

L'invention est illustrée par la description détaillée et les exemples qui suivent :

La composition selon l'invention comprend donc un composé de formule (I) qui est une molécule comprenant deux groupements principaux : d'une part un groupement phosphonate et d'autre part un groupement silanol ou précurseur de silanol. Ce composé est tel que la liaison P-C n'est pas hydrolysable *in vivo* et la liaison Si-C n'est pas hydrolysable *in vivo.*

Une véritable synergie entre les deux groupements est constatée, et conduit à obtenir un ciblage très spécifique du composé selon l'invention et une efficacité accrue de la fonction silanol au niveau de la cible.

Le groupement phosphonate va contribuer à véhiculer le composé selon l'invention au niveau des tissus notamment riches en phosphates, notamment l'os et le cartilage, et le groupement silanol va alors agir sur les métalloprotéases présentes dans la matrice extracellulaire.

Une autre fonction du groupement phosphonate est d'agir comme agent tampon au niveau de la cible.

Par ailleurs, du fait que la liaison P-C n'est pas hydrolysable *in vivo,* le groupement phosphonate n'est pas libéré au niveau du tissu. Il ne peut donc pas être incorporé par l'organisme dans la matrice osseuse et ne s'accumule pas dans l'os.

Les compositions pharmaceutiques selon l'invention sont donc particulièrement appréciées pour leur capacité à atteindre une cible spécifique, lieu d'une pathologie, et pour s'y distribuer de manière non-ubiquitaire au niveau de la matrice extra-cellulaire.

Suivant une variante avantageuse de l'invention, les compositions pharmaceutiques comprennent un composé de formule générale (I) dans laquelle R₄ et R₅ sont tous les deux indépendemment un atome d'hydrogène ou un groupement hydroxyle, et la somme (m + n) est strictement inférieure à 6. Il a en effet été constaté qu'une longue chaîne centrale peut représenter un facteur stérique susceptible de diminuer l'accessibilité du composé selon l'invention au site d'action. Par ailleurs, si la chaîne centrale est une succession de groupements apolaires, tels que notamment des groupements méthylène, la solubilité du composé selon l'invention peut diminuer fortement, ce qui le rend moins assimilable et de ce fait moins actif.

Suivant une autre variante avantageuse de l'invention, la composition pharmaceutique comprend un composé de formule générale (I) dans laquelle R₄ et R₅ sont chacun indépendamment un atome d'hydrogène ou un groupement hydroxyle et la somme (m + n) est égale à 2.

Une composition pharmaceutique préférée est la composition comprenant le diéthylphosphatoéthyltriéthoxysilane de formule

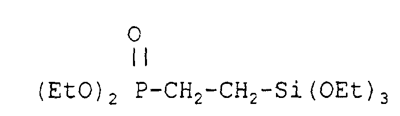

La composition pharmaceutique peut être administrée par voie orale, rectale ou topique. La voie orale est préférée ; elle se présente de préférence sous forme solide.

En effet, un but recherché est l'obtention d'une composition pharmaceutique comprenant un composé de formule (I) qui soit solide, unidose et administrable par voie orale, de manière à être utilisable en médication ambulatoire ne nécessitant pas la réalisation d'un acte médical préalable.

L'excipient approprié associé, dans la composition pharmaceutique, au composé de formule (I) peut être tout excipient thérapeutiquement acceptable. De préférence, l'excipient utilisé comprend de nombreuses fonctions hydroxyles. L'excipient préféré est le lactose.

Les compositions pharmaceutiques selon l'invention sont avantageusement utilisées pour l'obtention de médicaments destinés à soigner l'arthrose ou la polyarthrite chronique évolutive.

Les composés de formule générale (I) agissent, non pas de manière directe, mais de manière indirecte, au niveau de la matrice extracellulaire, comme agents inhibiteurs de métalloprotéases.

A titre d'exemple, l'utilisation des composés selon l'invention est étudiée sur l'activité des métalloprotéases telles que la collagénase, la stromélysine et la chymotrypsine, au niveau des chondrocytes, des ostéoblastes ou de l'épiderme.

On rappelle que les chondrocytes sont des cellules cartilagineuses présentes dans la matrice extracellulaire. Elles produisent des enzymes protéolytiques et notamment des métalloprotéases (stromélysine, collagénase,...) capables de digérer la matrice cartilagineuse. En situation normale, il y a un équilibre entre la chondrorésorption et de chondroformation. Une augmentation significative de l'activité des métalloprotéases crée un déséquilibre induisant une chondrorésorption massive. Cette suractivité des métalloprotéases est à l'origine de l'arthrose. Elle est notamment induite et réglée de façon autocrine et paracrine par des médiateurs de l'information, les cytokines, et plus particulièrement par l'interleukine-1 (IL-1) qui est une des cytokines les plus actives au cours de ce processus de dégradation du cartilage.

L'utilisation des composés de formule générale (I) selon l'invention, permet d'inhiber l'activité des métalloprotéases et plus particulièrement des sérines protéases, au niveau basal comme en situation de suractivation interleukine-dépendante (exemples 6 et 7).

On rappelle par ailleurs que la matrice extracellulaire d'un épiderme est décrite pour être un compartiment où se réalisent des réactions de protéolyse, dues, entre autres, à des enzymes de la famille des sérines protéases (trypsine, chymotrypsine, urokinase-like, desquamine) . Ces réactions d'hydrolyse sont impliquées dans l'activité catabolique de l'épiderme et dans plusieurs autres équilibres tels que l'hydratation, la desquamation ou l'inflammation.

On a notamment remarqué l'augmentation de l'activité de ces enzymes sur la peau après un stress oxydatif, tel que notamment un coup de soleil.

La desquamine est une sérine-protéase très proche de l'urokinase. On sait que cette enzyme est capable d'hydrolyser les desmosomes des kératinocytes et donc de moduler la desquamation de la peau. Cette enzyme serait aussi impliquée dans le contrôle de la libération d'IL-1 à partir de Pro-IL-1 fortement localisée dans les hémi-desmosomes (Forestier et Sauder 1988). Or, l'utilisation des composés de formule générale (I) selon l'invention, permet d'inhiber l'activité de la desquamine dans un extrait épidermique (exemple 10), donc de diminuer la libération d'IL-1 (responsable de l'inflammation et de la dégradation du cartilage) à partir de Pro-IL-1. Ainsi, les composés selon l'invention peuvent être valablement utilisés pour le traitement de pathologies chroniques, ainsi qu'à titre préventif.

Les exemples suivants servent à illustrer l'invention.

### Exemple 1 :

Effet d'un précurseur de diéthylphosphatoéthyltrisilanol, le diéthylphosphatoéthyltriéthoxysilane, sur l'activité de type collagénase d'une culture de chondrocytes humains
Des cultures de chondrocytes sont réalisées à partir de cartilages humains prélevés sur des cadavres dans les 12 heures qui suivent le décès. En milieu aqueux tamponné (pH 7,0 - 7,4), le diéthylphosphatoéthyltriéthoxysilane libère le diéthylphosphatoéthyltrisilanol correspondant, qui est le silanol actif. L'activité enzymatique de type collagénase de ces cultures de chondrocytes est quantifiée par la méthode de Cawston et Barrett. Sous l'effet du silanol une diminution dose-dépendante de l'activité "collagénase-like" des chondrocytes a été observée (30 % d'inhibition pour 500 *µ*g/ml de diéthylphosphatoéthyltrisilanol).

### Exemple 2 :

Effet d'un précurseur de diéthylphosphatoéthyltrisilanol, le diéthylphosphatoéthyltriéthoxysilane, sur l'activité de type collagénase de cultures de chondrocytes humains après induction à l'IL-1.
Des cultures de chondrocytes sont réalisées à partir de cartilages humains prélevés sur des cadavres dans les 12 heures qui suivent le décès. En milieu aqueux tamponné (pH 7,0 - 7,4), le diéthylphosphatoéthyltriéthoxysilane libère le diéthylphosphatoéthyltrisilanol correspondant, qui est le silanol actif. L'activité enzymatique de type collagénase de ces cultures de chondrocytes est quantifiée par la méthode de Cawston et Barrett après traitement préalable des cultures par l'IL-1. Sous l'effet du silanol une diminution dose-dépendante de l'activité "collagénase-like" des chondrocytes a été observée (21 % d'inhibition pour 500 *µ*g/ml de diéthylphosphatoéthyltrisilanol).

### Exemple 3 :

Effet d'un précurseur de diéthylphosphatoéthyltrisilanol, le diéthylphosphatoéthyltriéthoxysilane, sur l'activité de type stromélysine d'une culture de chondrocytes humains.
Des cultures de chondrocytes sont réalisées à partir de cartilages humains prélevés sur des cadavres dans les 12 heures qui suivent le décès. En milieu aqueux tamponné (pH 7,0 - 7,4), le diéthylphosphatoéthyltriéthoxysilane libère le diéthylphosphatoéthyltrisilanol correspondant, qui est le silanol actif. L'activité enzymatique de type stromélysine de ces cultures de chondrocytes est quantifiée par la méthode de digestion de la caséine couplée à la résorufine. Sous l'effet du silanol une diminution dose-dépendante de l'activité "stromélysine-like" des chondrocytes a été observée (37 % d'inhibition pour 500 µg/ml de diéthylphosphatoéthyltrisilanol)

### Exemple 4 :

Effet d'un précurseur de diéthylphosphatoéthyltrisilanol, le diéthylphosphatoéthyltriéthoxysilane, sur l'activité de type stromélysine d'une cultures de chondrocytes humains après induction à l'IL-1.
Des cultures de chondrocytes sont réalisées à partir de cartilages humains prélevés sur des cadavres dans les 12 heures qui suivent le décès. En milieu aqueux tamponné (pH 7,0 - 7,4), le diéthylphosphatoéthyltriéthoxysilane libère le diéthylphosphatoéthyltrisilanol correspondant, qui est le silanol actif. L'activité enzymatique de type stromélysine de ces cultures de chondrocytes est quantifiée, après traitement préalable des cultures avec de l'IL-1, par la méthode de digestion de la caséine couplée à la résorufine. Sous l'effet du silanol une diminution dose-dépendante de l'activité "stromélysine-like" des chondrocytes a été observée (18 % d'inhibition pour 500 *µ*g/ml de diéthylphosphatoéthyltrisilanol).

### Exemple 5 (de référence):

Effet d'un précurseur de diéthylphosphatoéthyltrisilanol, le diéthylphosphatoéthyltriéthoxysilane, sur l'activité "desquamine-like" d'un extrait épidermique
La desquamine est obtenue à partir d'extrait épidermique selon la méthode decrite par Brysk et al (1994). L'activité de la desquamine est évaluée par son aptitude à hydrolyser le substrat spécifique synthétique du tPA pour lequel elle a une très forte affinité. Après traitement d'un extrait épidermique par le silanol, une inhibition dose-dépendante de l'activité "desquamine-like" de l'extrait épidermique est observée. (25 % d'inhibition pour 300 *µ*g/ml de diéthylphosphatoéthyltrisilanol).

### Exemple 6 (de référence) :

Effet d'un précurseur de diéthylphosphatoéthyltrisilanol, le diéthylphosphatoéthyltriéthoxysilane, sur l'activité synthétique d'ostéoblastes.
Des cultures d'ostéoblastes sont réalisées à partir d'os humains prélevés sur des cadavres dans les 12 heures qui suivent le décès. En milieu aqueux tamponné (pH 7,0 - 7,4), le diéthylphosphatoéthyltriéthoxysilane libère du diéthylphosphatoéthyltrisilanol correspondant, qui est le silanol actif. L'activité synthétique des ostéoblastes est quantifiée par la mesure de l'activité de la phosphatase alcaline. Sous l'effet du diéthylphosphatoéthyltrisilanol, une augmentation dose-dépendante de l'activité des phosphatases alcalines des ostéoblastes a été observée (+ 24 % pour 100 µg/ml de diéthylphosphatoéthyltrisilanol).

### Exemple 7 (de référence) :

Effet d'un précurseur de diéthylphosphatoéthyltrisilanol, le diéthylphosphatoéthyltriéthoxysilane, sur l'activité IL-6 d'ostéoblastes.
Des cultures d'ostéoblastes sont réalisées à partir d'os humains prélevés sur des cadavres dans les 12 heures qui suivent le décès. En milieu aqueux tamponné (pH 7,0 - 7,4), le diéthylphosphatoéthyltriéthoxysilane libère du diéthylphosphatoéthyltrisilanol correspondant, qui est le silanol actif. L'IL-6 est quantifiée par la technique ELISA. Sous l'effet du diéthylphosphatoéthyltrisilanol une diminution dose-dépendante du taux d'IL-6 d'ostéoblastes a été observée (- 49 % pour 100 µg/ml de diéthylphosphatoéthyltrisilanol).

### Exemple 8 (de référence) :

Effet d'un précurseur de diéthylphosphatoéthyltrisilanol , le diéthylphosphatoéthyltriéthoxysilane, sur l'activité de type chymotrypsine d'un extrait épidermique.
Après prélèvement sur tissu cutané, l'épiderme est découpé puis mis en solution dans une solution tampon appropriée. La solution est homogénéisée à l'Ultra Turax (4 °C) jusqu'à obtention d'un extrait épidermique satisfaisant. L'activité chymotrypsine-like de cet extrait est estimé par la mesure de la dégradation de la N alpha-Benzoyl-L-Tyrosine Ethyl Ester (BTEE) (substrat synthétique spécifique). Sous l'effet du diéthylphosphatoéthyltrisilanol, une diminution dose-dépendante de l'activité "chymotrypsine-like" de l'extrait épidermique a été observée (27 % d'inhibition pour 400 µg/ml de diéthylphosphatoéthyltrisilanol).

## Revendications

1. Utilisation d'une composition pharmaceutique agissant par inhibition des sérines protéases et comprenant, en association avec tout excipient approprié, au moins un composé de formule générale (I) suivante :
R₆R₇P(O) - (CHR₅)ₘ - (CHR₄)ₙ - Si (OR₁) (OR₂) (R₃) (I)
dans laquelle :
R₁ et R₂ chacun indépendamment représentent un atome d'hydrogène ou un groupement alkyle,
R₃ représente un groupement hydroxyle ou un groupement alcoxy ou un groupement alkyle,
R₄ et R₅ représentent chacun indépendamment un atome d'hydrogène, un groupement hydroxyle, un groupement alkyle ou une fonction acide carboxylique,
R₆ et R₇ représentent chacun indépendamment un groupement alcoxy,
n est un entier supérieur ou égal à 0 et inférieur ou égal à 4
m est un entier strictement supérieur à 0 et inférieur ou égal à 3,
pour la préparation d'un médicament destiné à traiter l'arthrose ou la polyarthrite chronique évolutive.

2. Utilisation selon la revendication 1, **caractérisée en ce que** la composition comprend, en association avec tout excipient approprié, au moins un composé de formule (I) dans laquelle:
R₁ et R₂ chacun indépendamment représentent un atome d'hydrogène ou un groupement alkyle,
R₃ représente un groupement hydroxyle ou un groupement alcoxy ou un groupement alkyle,
R₄ et R₅ représentent chacun un atome d'hydrogène
R₆ et R₇ représentent chacun indépendamment un groupement alcoxy,
n est un entier supérieur ou égal à 0 et inférieur ou égal à 4
m est un entier strictement supérieur à 0 et inférieur ou égal à 3,
et la somme (m + n) est strictement inférieur à 6.

3. Utilisation selon l'une quelconque des revendications 1 ou 2, **caractérisée en ce que** la composition comprend au moins un composé de formule (I) dans laquelle
R₁ et R₂ chacun indépendamment représentent un atome d'hydrogène ou un groupement C₁₋₄ alkyle,
R₃ représente un groupement hydroxyle ou un groupement C₁₋₄ alcoxy ou un groupement C₁₋₄ alkyle,
R₄ et R₅ sont chacun indépendamment un atome d'hydrogène ou un groupement hydroxyle
R₆ et R₇ représentent chacun indépendamment un groupement C₁₋₄ alcoxy,
n est un entier supérieur ou égal à 0 et inférieur ou égal à 4
m est un entier strictement supérieur à 0 et inférieur ou égal à 3,
et la somme (m + n) est égale à 2.

4. Utilisation selon l'une quelconque des revendications 1 à 3, **caractérisée** en ce la composition comprend du diéthylphosphatoéthyltriéthoxysilane.

5. Utilisation selon la revendication 4, **caractérisée en ce que** le diéthylphosphatoéthyltriéthoxysilane libère du diéthylphosphatoéthyltrisilanol.

6. Utilisation selon l'une quelconque des revendications 1 à 5, **caractérisée en ce que** la composition est à administrer par voie orale, rectale ou topique.

7. utilisation selon l'une quelconque des revendications 1 à 6, **caractérisée en ce que** la composition est sous forme solide.

8. Utilisation selon l'une quelconque des revendications 1 à 7, **caractérisée en ce que** l'excipient comprend plusieurs fonctions hydroxyles.

9. Utilisation selon l'une des revendication 1 à 8, **caractérisée en ce que** l'excipient est le lactose.

10. Utilisation selon l'une des revendications 1, 8 ou 9, **caractérisée en ce que** le composé est le diéthylphosphatoéthyltrisilanol.

## Claims

1. Use of a pharmaceutical composition capable of inhibiting serine proteases and including, in association with any suitable excipient, at least one compound of the following general formula (I) :
R₆R₇P(O) - (CHR₅)m - (CHR₄)n - Si (OR₁) (OR₂) (R₃) (I)
in which :
R₁ and R₂ represent, each one independantly, a hydrogen atom or an alkyl group,
R₃ represents a hydroxyl group or an alcoxy group or an alkyl group,
R₄ and R₅ represent, each one independently, a hydrogen atom, a hydroxyl group, an alkyl group or a carboxylic acid function,
R₆ and R₇ represent, each one independently, an alcoxy group,
n is a whole number superior or equal to 0 and inferior or equal to 4,
m is a whole number strictly superior to 0 and inferior or equal to 3
for the preparation of a medication intended for the treatment of arthrosis or evolutive chronic polyarthritis.

2. Use according to the claim 1, **characterized in that** the composition includes, in association with any suitable excipient, at least one compound of the formula (I) in which :
R₁ and R₂ represent, each one independantly, a hydrogen atom or an alkyl group,
R₃ represents a hydroxyl group or an alcoxy group or an alkyl group,
R₄ and R₅ represent, each one, a hydrogen atom,
R₆ and R₇ represent, each one independently, an alcoxy group,
n is a whole number superior or equal to 0 and inferior or equal to 4,
m is a whole number strictly superior to 0 and inferior or equal to 3,
and the sum (m + n) is strictly inferior to 6.

3. Use according to any of the claims 1 or 2, **characterized in that** the composition includes at least one compound of the formula (I) in which :
R₁ and R₂ represent, each one independantly, a hydrogen atom or a C₁₋₄ alkyl group,
R3 represents a hydroxyl group or a C₁₋₄ alcoxy group or a C₁₋₄ alkyl group,
R₄ and R₅ represent, each one independently, a hydrogen atom or a hydroxyl group,
R₆ and R₇ represent, each one independently, a C₁₋₄ alcoxy group,
n is a whole number superior or equal to 0 and inferior or equal to 4,
m is a whole number strictly superior to 0 and inferior or equal to 3.
and the sum (m + n) is strictly equal to 2.

4. Use according to any of the claims 1 to 3, **characterized in that** the composition includes diethylphosphatoethyltriethoxysilane.

5. Use according to the claim 4, **characterized in that** the diethylphosphatoethyltriethoxysilane releases the diethylphosphatoethyltrisilanol.

6. Use according to any of the claims 1 to 5, **characterized in that** the composition is to be administered orally, rectally or topically.

7. Use according to any of the claims 1 to 6, **characterized in that** the composition is under solid form.

8. Use according to any of the claims 1 to 7, **characterized in that** the excipient bears several hydroxyl functions.

9. Use according to any of the claims 1 to 8, **characterized in that** the excipient is lactose.

10. Use according to any of the claims 1, 8 or 9, **characterized in that** the compound is diethylphosphatoethyltrisilanol.

## Patentansprüche

1. Die Verwendung einer pharmazeutischen Zusammensetzung, die durch die Inhibition von Serin-Proteasen wirkt, und die zusammen mit jedem beliebigen geeigneten Exzipienten mindestens eine Verbindung mit der folgenden allgemeinen Formel (I) umfasst:
R₆R₇P(O) - (CHR₅)ₘ - (CHR₄)ₙ- Si (OR₁)(OR₂)(R₃) (I)
wobei:
R₁ und R₂ unabhängig voneinander jeweils ein Wasserstoffatom oder eine Alkylgruppe darstellen,
R₃ eine Hydroxylgruppe oder eine Alkoxygruppe oder eine Alkylgruppe darstellt,
R₄ und R₅ unabhängig voneinander jeweils ein Wasserstoffatom, eine Hydroxylgruppe, eine Alkylgruppe oder eine Carbonsäurefunktion darstellen,
R₆ und R₇ unabhängig voneinander jeweils eine Alkoxygruppe darstellen,
n eine ganze Zahl ist, die größer oder gleich 0 und kleiner oder gleich 4 ist,
m eine ganze Zahl ist, die genau größer als 0 und kleiner oder gleich 3 ist,
für die Zubereitung eines Medikaments, das zur Behandlung von Arthrose oder rheumatischer Polyarthritis bestimmt ist.

2. Verwendung gemäß Anspruch 1, **dadurch gekennzeichnet, dass** die Zusammensetzung zusammen mit jedem beliebigen geeigneten Exzipienten mindestens eine Verbindung mit der Formel (I) umfasst, wobei:
R₁ und R₂ unabhängig voneinander jeweils ein Wasserstoffatom oder eine Alkylgruppe darstellen,
R₃ eine Hydroxylgruppe oder eine Alkoxygruppe oder eine Alkylgruppe darstellt,
R₄ und R₅ jeweils ein Wasserstoffatom darstellen,
R₆ und R₇ unabhängig voneinander jeweils eine Alkoxygruppe darstellen,
n eine ganze Zahl ist, die größer oder gleich 0 und kleiner oder gleich 4 ist,
m eine ganze Zahl ist, die streng größer als 0 und kleiner oder gleich 3 ist
und die Summe (m + n) genau kleiner als 6 ist.

3. Verwendung gemäß Anspruch 1 oder 2, **dadurch gekennzeichnet, dass** die Zusammensetzung mindestens eine Verbindung der Formel (I) umfasst, wobei
R₁ und R₂ unabhängig voneinander jeweils ein Wasserstoffatom oder eine C₁₋₄ Alkylgruppe darstellen,
R₃ eine Hydroxylgruppe oder eine C₁₋₄ Alkoxygruppe oder eine C₁₋₄ Alkylgruppe darstellt,
R₄ und R₅ unabhängig voneinander jeweils ein Wasserstoffatom oder eine Hydroxylgruppe darstellen,
R₆ und R₇ unabhängig voneinander jeweils eine C₁₋₄ Alkoxygruppe darstellen,
n eine ganze Zahl ist, die größer oder gleich 0 und kleiner oder gleich 4 ist,
m eine ganze Zahl ist, die genau größer als 0 und kleiner oder gleich 3 ist
und die Summe (m + n) gleich 2 ist.

4. Verwendung gemäß einem der Ansprüche 1 bis 3, **dadurch gekennzeichnet, dass** die Zusammensetzung Diethylphosphatethyltriethoxysilan umfasst.

5. Verwendung gemäß Anspruch 4, **dadurch gekennzeichnet, dass** Diethylphosphatethyltriethoxysilan Diethylphosphatethyltrisilanol freisetzt.

6. Verwendung gemäß einem der Ansprüche 1 bis 5, **dadurch gekennzeichnet, dass** die Zusammensetzung oral, rektal oder topisch zu verabreichen ist.

7. Verwendung gemäß einem der Ansprüche 1 bis 6, **dadurch gekennzeichnet, dass** die Zusammensetzung in fester Form vorhanden ist.

8. Verwendung gemäß einem der Ansprüche 1 bis 7, **dadurch gekennzeichnet, dass** der Exzipient mehrere Hydroxylfunktionen umfasst.

9. Verwendung gemäß einem der Ansprüche 1 bis 8, **dadurch gekennzeichnet, dass** der Exzipient Laktose ist.

10. Verwendung gemäß einem der Ansprüche 1, 8 oder 9, **dadurch gekennzeichnet, dass** die Verbindung Diethylphosphatethyltrisilanol ist.
